(19) Europäisches Patentamt
European Patent Office
Office européen des brevets

(11) Veröffentlichungsnummer: **0 098 568 B1**

(12) # EUROPÄISCHE PATENTSCHRIFT

(45) Veröffentlichungstag der Patentschrift: **28.08.91**

(51) Int. Cl.$^5$: **A61K 9/06**, A61K 9/10

(21) Anmeldenummer: **83106512.3**

(22) Anmeldetag: **04.07.83**

Die Akte enthält technische Angaben, die nach dem Eingang der Anmeldung eingereicht wurden und die nicht in dieser Patentschrift enthalten sind.

(54) **Kortikoidhaltige Zubereitungen zur topischen Applikation.**

(30) Priorität: **07.07.82 DE 3225849**

(43) Veröffentlichungstag der Anmeldung:
**18.01.84 Patentblatt 84/03**

(45) Bekanntmachung des Hinweises auf die Patenterteilung:
**28.08.91 Patentblatt 91/35**

(84) Benannte Vertragsstaaten:
**AT BE CH DE FR GB IT LI LU NL SE**

(56) Entgegenhaltungen:
**EP-A- 0 042 827**

**SEIFEN, OLE, FETTE, WASCHE, Band 106, Nr.20, 11 Dezember 1980, AUGSBURG (DE)**

(73) Patentinhaber: **SCHERING AKTIENGESELL-SCHAFT Berlin und Bergkamen**
**Müllerstrasse 170/178 Postfach 65 03 11**
**W-1000 Berlin 65(DE)**

(72) Erfinder: **Stindl, Wolfgang**
**Kleinhöfleiner Hauptstrasse 24**
**A-7000 Eisenstadt(AT)**
Erfinder: **Zimmermann, Ingfried, Dr.**
**1 Berlin 28**
**Gollanczstrasse 28 c(DE)**
Erfinder: **Reckers, Renate, Dr.**
**1 Berlin 62**
**Kufsteiner Strasse 2(DE)**
Erfinder: **Wendt, Hans, Dr.**
**1 Berlin 38**
**Ernst-Ring-Strasse 14(DE)**
Erfinder: **Arndt, Rainold Dr.**
**1 Berlin 45**
**Undinestrasse 8(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft die in den Patentansprüchen 1 bis 6 beanspruchte kortikoidhaltige Zubereitung zur topischen Applikation, sowie das in den Patentansprüchen 7 und 8 gekennzeichnete Verfahren zu deren Herstellung.

Grundsätzlich eignet sich jedes antiinflammatorisch wirksame Kortikoid zur Herstellung der erfindungsgemäßen kortikoidhaltigen Zubereitung. Bevorzugt geeignet sind hierzu aber die in den Patentansprüchen 2 bis 6 gekennzeichneten Kortikoide.

Kortikoide der allgemeinen Formel I gemäß Patentanspruch 1, die sich zur Herstellung der erfindungsgemäßen kortikoidhaltigen Zubereitungen eignen, sind Hydrocortison, Prednisolon, 6α-Methylhydrocortison, 6α-Methylprednisolon und deren Ester oder Acetale, wie zum Beispiel das Hydrocortison-21-acetat, das Hydrocortison-17-butyrat, das Hydrocortison-17-valerianat, das Prednisolon-21-acetat, das Prednisolon-17-valerianat, das 6α-Methylhydrocortison-21-acetat, das 6α-Methylprednisolon-21-acetat, das 6α-Methylhydr ocortison-17-butyrat-21-acetat, das 6α-Methylprednisolon-21-acetat-propionat, das 6α-Methylhydrocortison-17,21-dipropionat oder das 21-Acetoxy-17α-ethoxymethoxy-11β-hydroxy-1,4-pregnadien-3,20-dion.

Die erfindungsgemäße Zubereitung enthält 0,005 bis 2 und vorzugsweise 0,05 bis 0,5 Gewichtsprozent Kortikoid; die Konzentration derselben ist selbstverständlich von der relativen Wirksamkeit des Kortikoids abhängig.

Wie bei den vorbekannten kortikoidhaltigen Zubereitungen zur topischen Applikation kann auch die erfindungsgemäße Zubereitung in Form einer Öl/Wasser-Emulsion (zum Beispiel als Milch oder Creme) in Form einer Wasser/Öl-Emulsion (zum Beispiel als Fettsalbe) oder als ölige Suspension (zum Beispiel als Fettspray) vorliegen. Bei den Öl/Wasser-Emulsionen oder Wasser/Öl-Emulsionen verwendet man erfindungsgemäß 3 bis 15 Gewichtsprozent Jojoba-Öl. Das Jojoba-Öl wird vorzugsweise in gereinigter Form (zum Beispiel Puroba-Öl) verwendet.

Die Öl/Wasser-Emulsionen und die Wasser/Öl-Emulsionen können in konventioneller Weise unter Verwendung konventioneller Emulgatoren hergestellt werden (Kirk Othmer: Enzyclopedia of Chemical Technology, 3. Auflage, 1979; John Wiley & Sons; New York etc. Vol 8, Seite 900 bis 930 und Dr. Otto-Albrecht Neumüller: Römpps Chemie Lexikon 7. Auflage, 1973; Franck'sche Verlagshandlung Stuttgart, Seite 1009 bis 1013). Die für diese Emulsionen verwendeten Wachse, Emulgatoren und übrigen Zusätze sind die gleichen, wie man sie konventioneller Weise bei emulgierten Hautpflegemitteln verwendet (Dr. Otto-Albrecht Neumüller: Römpps Chemie Lexikon, 7. Auflage 1973; Franck'sche Verlagshandlung Stuttgart, Seite 1427 und 1428).

Eine in der erfindungsgemäßen Zubereitung eingesetzte Öl/Wasser-Emulsion kann aus hydrophilen und/oder lipophilen Wirkstoffen, Fettphase, Öl/Wasser-Emulgator, wässriger Phase und Konservierungsmittel bestehen.

Als hydrophile und/oder lipophile Wirkstoffe können Feuchthaltefaktoren (Hydrokomplexe), wie zum Beispiel Glycerin, Polyäthylenglykole oder Aminosäuregemische, Puroba-Oil (= Jojoba-Öl), Vitamine (vorzugsweise Vitamin A und E) Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eignen sich Kohlenwasserstoffe wie zum Beispiel Vaseline, Paraffine oder Stearin, oder Wachse wie zum Beispiel Bienenwachs. Geeignete Öl/Wasser-Emulgatoren sind beispielsweise Stearylalkohol, Polyoxyäthylenstearate (wie zum Beispiel MYRJ(R)), Komplexemulgatoren (wie zum Beispiel Amphoterin(R)) und Sorbitanfettsäureester (wie zum Beispiel Span(R)) oder Carboxyvinylpolymerisate (wie zum Beispiel Carbopol(R)). Die wässrige Phase kann zusätzlich noch Puffersubstanzen, wie zum Beispiel das Dinatriumsalz der Äthylendiamin-N,N,N',N'-tetraessigsäure und Konservierungsmittel wie Chlorquinaldol, Parabene oder Benzalkoniumchlorid enthalten.

In der Öl/Wasser-Emulsion beträgt der Anteil der inneren Emulsion vorzugsweise 30 bis 49 Gew. %, die Teilchengröße der inneren Emulsion liegt vorzugsweise zwischen 1 $\mu$m und 50 $\mu$m.

Eine im erfindungsgemäßen kosmetischen Mittel einsetzbare Wasser/Öl-Emulsion besteht wiederum aus hydrophilen und/oder lipophilen Wirkstoffen, wie sie auch in der Öl/Wasser-Emulsion verwendet werden, Fettphase, Wasser/Öl-Emulgator und wässriger Phase. Als ölige Phase oder Fettphase der Wasser/Öl-Emulsion können Kohlenwasserstoffe, z.B. Paraffine und Vaseline, synthetische, pflanzliche und tierische Öle bzw. Wachse (wie zum Beispiel Olivenöl, Erdnußöl, feines Knochen-öl, Mandelöl, Lanolin, Bienenwachs oder Sonnenblumenöl) eingesetzt werden, als wässrige Phase gereinigtes demineralisiertes Wasser und als Wasser/Öl-Emulgator Wollfett (=Lanolin), Fettalkohole wie zum Beispiel Cetylalkohol, Myristylalkohol, Stearylalkohol oder Cerylalkohol, Fettsäureester, wie zum Beispiel Bienenwachs (Cera alba) oder Wachsalkoholester oder Mischester (wie zum Beispiel Dehymuls(R)).

In der Wasser/Öl-Emulsion beträgt der Anteil der inneren Emulsion vorzugsweise 30 bis 49 Gew. %, die

Teilchengröße der inneren Emulsion liegt vorzugsweise zwischen 1 μm und 100 μm. Bei der Dispergierung beider Phasen wird sie nochmals zerkleinert und liegt im fertigen Produkt unterhalb von 50 μm.

Die feindispersen Systeme werden zusätzlich noch mit mikronisiertem Kortikoid (Korngröße vorwiegend 1 bis 20 μm) und Duftstoffen, wie zum Beispiel diejenige der Crematest(R) -Serie versetzt, und bis zur gleichmäßigen Verteilung derselben gerührt.

Die Erfindung betrifft insbesondere eine kortikoidhaltige Zubereitung in Form einer Salbe oder Creme, die dadurch gekennzeichnet ist, daß sie die hydrophilen und/oder lipophilen Wirkstoffe, die Fettphase und wässrige Phase in Form einer feindispersen Mischung einer einen Öl/Wasser-Emulgator und Konservierungsmittel enthaltenden Öl/Wasser-Emulsion und einer einen Wasser/Öl-Emulgator enthaltenden Wasser/Öl-Emulsion enthält. Öl/Wasser-Emulsion und Wasser/Öl-Emulsion werden wie bereits beschrieben hergestellt.

Mit Hilfe der die feindisperse Mischung der beiden Emulsionstypen enthaltenden Creme kann auf der Haut je nach ihrer Feuchtigkeit auf ihren unterschiedlichen Bereichen bzw. je nach Hauttypus ein entsprechender Öl/Wasser- oder Wasser/Öl-Hydrolipidfilm erzeugt werden.

Erfindungsgemäß beträgt die Teilchengröße der Emulsion 2 bis 50 μm.

Die Erfindung betrifft insbesondere auch ein Verfahren zur Herstellung einer kortikoidhaltigen Zubereitung in Form einer Salbe, Paste oder Creme, das dadurch gekennzeichnet ist, daß man aus Fettphase, wässriger Phase, Öl/Wasser-Emulgator und Konservierungsmittel eine Öl/Wasser-Emulsion und aus Fettphase, wässriger Phase und Wasser/Öl-Emulgator eine Wasser/Öl-Emulsion herstellt und daß man die beiden Emulsionen unter Zusatz von hydrophilen und/oder lipophilen Wirkstoffen unter Vakuum bei einer Temperatur zwischen 20 und 40° C innig verrührt und darauf die erhaltene feindisperse Mischung mit dem mikronisiertem Kortikoid (Teilchengröße vorwiegend 1 bis 20 μm ) und mit Duftstoffen versetzt und bis zur gleichmäßigen Verteilung derselben rührt.

Wenn nun die beiden Emulsionstypen bei Temperaturen zwischen 20 und 40° C, vorzugsweise bei 30° C unter Vakuum besonders schonend, dessen ungeachtet jedoch sehr intensiv, in einen Rührwerksbehälter vereinigt werden, sodaß schließlich eine sehr feine Dispersion der beiden Emulsionen, deren Teilchengröße zwischen 2 und 50 μm, vorzugsweise zwischen 5 und 15 μm beträgt erzielt wird, dann bleiben die beiden Emulsions typen unverändert nebeneinander in der hergestellten Creme erhalten. Dieses Emulsionssystem bleibt bei erheblicher Verdünnung, z. B. mit Flüssigphase, stabil. Es wird angenommen, daß für die Stabilität dieses Emulsionssystems die Teilchengröße ebenso wie das Zusammenwirken der Emulgatoren von Bedeutung ist. Wesentlich ist, daß bei der Herstellung der erfindungsgemäßen kortikoidhaltigen Zubereitung die zum Einsatz kommenden Emulsionen dispergiert und nicht homogenisiert werden.

Die Vereinigung der beiden Emulsionen erfolgt vorzugsweise unter einem Vakuum von 67 Pa bis 6700 Pa (0,5-50 Torr). Die Rührgeschwindigkeit ist, wie dem Fachmann wohlbekannt ist, von dem verwendeten Rührwerk abhängig und muß in an sich bekannter Weise ermittelt werden.

Das Mischungsverhältnis von Öl/Wasser-Emulsion und Wasser/Öl-Emulsion liegt vorzugsweise bei 20 bis 80 Gew. % und insbesondere bei 35 % bis 65 % Öl/Wasser-Emulsion.

Das feindisperse System wird zusätzlich noch mit Duftstoffen, wie zum Beispiel diejenige der Crematest(R)-Serie versetzt.

Die erfindungsgemäße kortikoidhaltige Zubereitung in Form einer Creme kann beispielsweise folgende Zusammensetzung haben:

| | | Toleranzen |
|---|---|---|
| Hydrocortison-21-acetat mikronisiert (Teilchengröße vorwiegend 1 bis 20μm) | 0,5 % | 0,1 - 1 % |
| Jojobaöl (Pur-oba-Oil) | 5 % | 5 - 10 % |
| Cera-alba (Bienenwachs) | 1 % | 1 - 5 % |
| Dehymuls(R) | 1 % | 1 - 3 % |
| Stearylalkohol | 4 % | 4 - 8 % |
| Kohlenwasserstoffe | 30 % | 30 - 50 % |
| Carbopol(R) | 1 % | |
| MYRJ(R) | 3 % | 2 - 5 % |
| Dinatriumedetat | 1 % | |
| Chlorquinaldol | 1 % | |
| gereinigtes demineralisiertes Wasser | 52 % | 30 - 55 % |
| Parfumöl | 0,5 % | |

Die Angaben sind in Gewichtsprozent aufgeführt.

Die nachfolgenden Ausführungsbeispiele dienen zur Erläuterung des erfindungsgemäßen Verfahrens, welches mittels allen Vorrichtungen durchgeführt werden kann, die man konventioneller Weise zur Herstellung von Salben, Cremes etc. anwendet.

Beispiel 1

Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB ist die Abkürzung für das Deutsche Arzneibuch, amtliche Ausgabe, 8. Auflage 1978 - 40,00 g Stearylalkohol, 30,00 g MYRJ(R) und 50,00 g Puroba-Öl eingetragen. Man rührt die Mischung noch solange, bis eine Emulsion mit einer Teilchengröße von 20-70 μm entsteht.

Herstellung der Wasser/Öl-Emulsion.

228,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch solange, bis eine Emulsion mit einer Teilchengröße von 20-70 μm entsteht.

Herstellung einer Creme.

Die Wasser/Öl-Emulsion wird unter kräftigem Rühren unter einem Vakuum von 1330 Pa (10 Torr) in die Öl/Wasser-Emulsion eingetragen. Man rührt noch solange, bis eine Dispersion mit einer Teilchengröße von 10 bis 50μm entsteht, entfernt das Vakuum, setzt unter Rühren noch 5,000 g Hydrocortison-21-acetat - mikronisiert Teilchengröße vorwiegend 1 bis 20μm und 2,00 g eines Duftstoffes der Crematest(R)-Serie zu und rührt, bis sich beide Komponenten in der Salbengrundlage gleichmäßig verteilt haben.

Beispiel 2

Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB ist die Abkürzung für das Deutsche Arzneibuch, amtliche Ausgabe, 8. Auflage 1978 - 40,00 g Stearylalkohol, 30,00 g MYRJ(R) und 50,00 g Puroba-Öl eingetragen. Man rührt die Mischung noch solange, bis eine Emulsion mit einer Teilchengröße von 20-70 $\mu$m entsteht.

Herstellung der Wasser/Öl-Emulsion.

227,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch solange, bis eine Emulsion mit einer Teilchengröße von 20-70$\mu$m entsteht.

Herstellung einer Creme.

Die Wasser/Öl-Emulsion wird unter kräftigem Rühren unter einem Vakuum von 1330 Pa in die Öl/Wasser-Emulsion eingetragen. Man rührt noch solange, bis eine Dispersion mit einer Teilchengröße von 10 bis 50 $\mu$m entsteht, entfernt das Vakuum, setzt unter Rühren noch 1,000 g 21-Acetoxy-11$\beta$-hydroxy-6$\alpha$-methyl-17$\alpha$-propionyloxy-1,4-pregnadien-3,20-dion - mikronisiert Teilchengröße vorwiegend 1 bis 20$\mu$m und 2,00 g eines Duftstoffes der Crematest(R)-Serie zu und rührt, bis sich beide Komponenten in der Salbengrundlage gleichmäßig verteilt haben.

Beispiel 3

Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB ist die Abkürzung für das Deutsche Arzneibuch, amtliche Ausgabe, 8. Auflage 1978 - 40,00 g Stearylalkohol, 30,00 g MYRJ(R) und 50,00 g Puroba-Öl eingetragen. Man rührt die Mischung noch solange, bis eine Emulsion mit einer Teilchengröße von 20-70$\mu$m entsteht.

Herstellung der Wasser/Öl-Emulsion.

227,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch solange, bis eine Emulsion mit einer Teilchengröße von 20-70$\mu$m entsteht.

Herstellung einer Creme.

Die Wasser/Öl-Emulsion wird unter kräftigem Rühren unter einem Vakuum von 1330 Pa (10 Torr)in die Öl/Wasser-Emulsion eingetragen. Man rührt noch solange, bis eine Dispersion mit einer Teilchengröße von 10 bis 50 $\mu$m entsteht, entfernt das Vakuum, setzt unter Rühren noch 1,000 g 21-Acetoxy-17$\alpha$-butyryloxy-11$\beta$-hydroxy-6$\alpha$-methyl-4-pregnen-3,20-dion - mikronisiert Teilchengröße vorwiegend 1 bis 20 $\mu$m und 2,00 g eines Duftstoffes der Crematest(R)-Serie zu und rührt bis sich beide Komponenten in der Salbengrundlage gleichmäßig verteilt haben.

## Patentansprüche

1. Kortikoidhaltige Zubereitung zur topischen Applikation, enthaltend 0,005 bis 2 Gewichtsprozent eines Kortikoids der allgemeinen Formel I

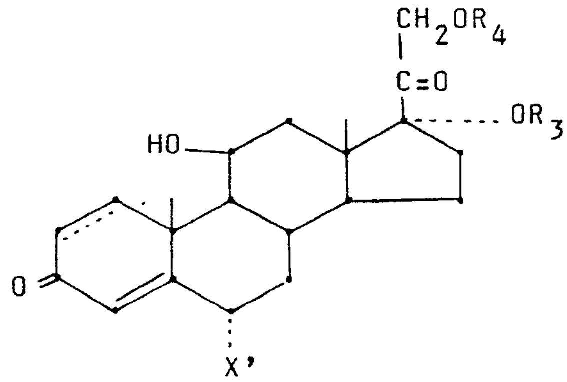

(I),

worin

| | |
|---|---|
| ..... | eine Einfachbindung oder eine Doppelbindung, |
| X | ein Wasserstoffatom oder eine Methylgruppe bedeutet, und worin |
| $R_3$ und $R_4$ | gleich oder verschieden sind und Wasserstoff, 2 bis 6 Kohlenstoffatome enthaltende Alkanoylgruppen, |

oder Benzoylgruppen darstellen, dadurch gekennzeichnet, daß sie 3 bis 15 Gewichtsprozent Jojoba-Öl enthält.

2. Kortikoidhaltige Zubereitung gemäß Patentanspruch 1 , dadurch gekennzeichnet, daß sie 3 bis 10 Gewichtsprozent Jojoba-Öl enthält.

3. Kortikoidhaltige Zubereitung gemäß Patentanspruch 1 und 2, dadurch gekennzeichnet, daß sie mittels Aktivkohle entfärbtes und gereinigtes Jojoba-Öl enthält.

4. Kortikoidhaltige Zubereitung gemäß Patentanspruch 1 bis 3, dadurch gekennzeichnet, daß sie als Träger- und Hilfsstoffe eine Fettphase, eine wässrige Phase, Emulgatoren, Konservierungsmittel sowie gegebenenfalls zusätzlich Duftstoffe und weitere hydrophile und/oder hydrophobe Wirkstoffe enthält.

5. Kortikoidhaltige Zubereitung gemäß Patentanspruch 4, dadurch gekennzeichnet, daß sie die hydrophilen und/oder lipophilen Wirkstoffe, die Fettphase und wässrige Phase in Form einer feindispersen Mischung einer einen Öl/ Wasser-Emulgator und Konservierungsmittel enthaltenden Öl/Wasser-Emulsion und einer einen Wasser/Öl-Emulgator enthaltenden Wasser/Öl-Emulsion enthält.

6. Kortikoidhaltige Zubereitung gemäß Patentanspruch 5, dadurch gekennzeichnet, daß die Teilchengröße der Emulsionen 2 bis 50 $\mu$m beträgt.

7. Verfahren zur Herstellung einer kortikoidhaltigen Zubereitung gemäß Patentansprüchen 1 bis 6, dadurch gekennzeichnet, daß man aus Fettphase, wässriger Phase, Öl/Wasser-Emulgator und Konservierungsmittel eine Öl/Wasser-Emulsion und aus Fettphase, wässriger Phase und Wasser/Öl-Emulgator eine Wasser/Öl-Emulsion herstellt und daß man die beiden Emulsionen unter Zusatz von hydrophilen und/oder lipophilen Wirkstoffen unter Vakuum bei einer Temperatur zwischen 20 und 40° C innig verrührt und darauf die erhaltene feindisperse Mischung mit dem mikronisierten Kortikoid und mit Duftstoffen versetzt.

8. Verfahren zur Herstellung einer kortikoidhaltigen Zubereitung gemäß Patentanspruch 7, dadurch gekennzeichnet, daß man aus den Wirkstoffen, den Träger- und Hilfsstoffen eine Emulsion mit einer Teilchengröße von 1 bis 50 $\mu$m herstellt.

**Claims**

1. Corticoid-containing preparation for topical application containing from 0.005 to 2 % by weight of a corticoid of the general formula I

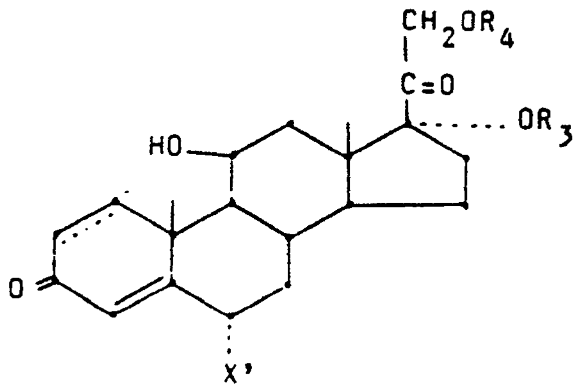

(I)

wherein

| | |
|---|---|
| ..... | represents a single bond or a double bond, |
| X | represents a hydrogen atom or a methyl group, and wherein |
| $R_3$ and $R_4$ | are the same or different and represent hydrogen, alkanoyl groups containing from 2 to 6 carbon atoms, or benzoyl groups, |

characterised in that it contains from 3 to 15 % by weight jojoba oil.

2. Corticoid-containing preparation according to patent claim 1, characterised in that it contains from 3 to 10 % by weight jojoba oil.

3. Corticoid-containing preparation according to patent claims 1 and 2, characterised in that it contains jojoba oil that has been decolorised and purified by means of activated carbon.

4. Corticoid-containing preparation according to patent claims 1 to 3, characterised in that it contains, as carriers and auxiliaries, a fatty phase, an aqueous phase, emulsifiers, preservatives and, optionally, in addition perfumes and other hydrophilic and/or hydrophobic active substances.

5. Corticoid-containing preparation according to patent claim 4, characterised in that it contains the hydrophilic and/or lipophilic active substances, the fatty phase and the aqueous phase in the form of a finely dispersed mixture of an oil-in-water emulsion that contains an oil/water emulsifier and preservatives, and a water-in-oil emulsion that contains a water/oil emulsifier.

6. Corticoid-containing preparation according to patent claim 5, characterised in that the particle size of the emulsions is from 2 to 50 $\mu$m.

7. Process for the production of a corticoid-containing preparation according to patent claims 1 to 6, characterised in that an oil-in-water emulsion is produced from fatty phase, aqueous phase, oil/water emulsifier and preservatives, and a water-in-oil emulsion is produced from fatty phase, aqueous phase and water/oil emulsifier, and the two emulsions are intimately stirred, with the addition of hydrophilic and/or lipophilic active substances, at a temperature of from 20 to 40 °C in vacuo, and there are subsequently added to the resulting finely dispersed mixture the micronised corticoid and perfumes.

8. A process for the production of a corticoid-containing preparation according to patent claim 7, characterised in that an emulsion having a particle size of from 1 to 50 $\mu$m is produced from the active substances, the carriers and the auxiliaries.

## Revendications

1. Composition corticoïdique destinée à être appliquée localement, qui contient de 0,005 à 2% en poids d'un corticoïde répondant à la formule générale I :

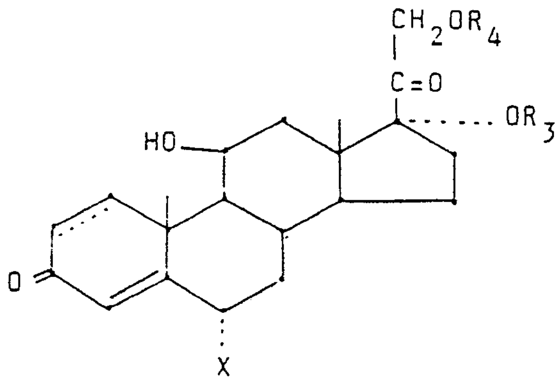

(I)

dans laquelle

..... représente une liaison simple ou une liaison double,

$\overline{X}$ représente un atome d'hydrogène ou un radical méthyle, et

$R_3$ et $R_4$ représentent chacun, indépendamment l'un de l'autre, l'hydrogène, un radical alcanoyle contenant de 2 à 6 atomes de carbone, ou un radical benzoyle,

et qui est caractérisée en ce qu'elle contient de 3 à 15% en poids d'huile de jojoba.

2. Composition corticoïdique selon la revendication 1 caractérisée en ce qu'elle contient de 3 à 10% en poids d'huile de jojoba.

3. Composition corticoïdique selon l'une des revendications 1 et 2, caractérisée en ce qu'elle contient une huile de jojoba qui a été purifiée et décolorée par du charbon actif.

4. Composition corticoïdique selon l'une quelconque des revendications 1 à 3, caractérisée en ce qu'elle contient, comme excipients et adjuvants, une phase grasse, une phase aqueuse, des émulsionnants, des conservateurs et en outre, éventuellement, des parfums et d'autres substances actives hydrophiles et/ou hydrophobes.

5. Composition corticoïdique selon la revendication 4 caractérisée en ce qu'elle contient les susbtances actives hydrophiles et/ou lipophiles, la phase grasse et la phase aqueuse sous la forme d'un mélange finement dispersé d'une émulsion LH (huile-dans-l'eau) contenant un émulsionnant huile/eau et un conservateur, et d'une émulsion HL (eau-dans-l'huile) contenant un émulsionnant eau/huile.

6. Composition corticoïdique selon la revendication 5 caractérisée en ce que la dimension des particules des émulsions est comprise entre 2 et 50 $\mu$m.

7. Procédé pour préparer une composition corticoïdique selon l'une quelconque des revendications l à 6, procédé caractérisé en ce qu'on prépare une émulsion LH à partir d'une phase grasse, d'une phase aqueuse, d'un émulsionnant huile/eau et d'un conservateur, et une émulsion HL à partir d'une phase grasse, d'une phase aqueuse et d'un émulsionnant eau/huile, on mélange intimement les deux émulsions tout en y ajoutant des substances actives hydrophiles et/ou lipophiles, à une température de 20 à 40 $^\circ$C et sous pression réduite, puis on ajoute au mélange finement dispersé obtenu, le corticoïde micronisé et des parfums.

8. Procédé pour préparer une composition corticoïdique selon la revendication 7 caractérisé en ce que, à partir des substances actives, des excipients et des adjuvants, on prépare une émulsion ayant une dimension des particules comprise entre 1 et 50 $\mu$m.